# FASCICULE DE BREVET EUROPEEN

(11) **EP 3 089 615 B1**
(45) Date de publication et mention de la délivrance du brevet: **10.04.2019**
(21) Numéro de dépôt: 14827827.8
(22) Date de dépôt: 12.12.2014
(51) Int. Cl.: A45D 33/00, A45D 33/18, A45D 40/16, A45D 40/24

(54) **ASSEMBLAGE COSMÉTIQUE COMPRENANT UN PRODUIT COULE**
KOSMETIKEINHEIT MIT EINEM GEGOSSENEN PRODUKT
COSMETIC ASSEMBLY INCLUDING A POURED PRODUCT

(30) Priorité: 31.12.2013 FR 1363742
(43) Date de publication de la demande: 09.11.2016
(73) Titulaire: LVMH RECHERCHE, 45800 St. Jean de Braye (FR)
(72) Inventeur: PERRIN, Lily Ann, F-45190 Beaugency (FR)
(74) Mandataire: Chantraine, Sylvie Hélène
(86) Numéro de dépôt international: PCT/FR2014/053313
(87) Numéro de publication internationale: WO 2015/101729

(56) Documents cités:
- FR-A1- 2 956 833
- US-A- 6 058 942

## Description

L'invention concerne le domaine des produits cosmétiques, destinés notamment au soin ou au maquillage du visage, qui sont constitués de plusieurs compositions solides différentes. La présente invention se rapporte en particulier à un produit cosmétique proposant plusieurs compositions de couleurs et/ou de formes différentes dans un même récipient.

Le produit cosmétique de l'invention présente l'originalité de comprendre à la fois une composition qui a été moulée ou pressée, et une composition qui a été coulée à chaud dans le récipient.

### ART ANTERIEUR

Les boîtiers utilisés pour la présentation simultanée de plusieurs compositions cosmétiques sont généralement des emballages à fond plat, leur fonction étant de présenter en toute occasion à l'utilisateur une surface importante de produit pour faciliter le choix de la teinte (en évitant de devoir ouvrir plusieurs boîtiers différents), pour faciliter le prélèvement à l'aide du doigt ou d'un applicateur, et pour donner un aspect attrayant au produit. Cette surface est généralement sensiblement plane.

Les boîtiers de l'art antérieur comprennent généralement deux parties : un couvercle et un réceptacle articulés autour d'une charnière. Le réceptacle est généralement composé de plusieurs compartiments solidaires du boîtier contenant chacun des teintes différentes.

D'une manière générale, les boîtiers de l'art antérieur comprennent un couvercle en creux qui peut être muni d'un miroir intérieur et une base en creux, qui peut être munie d'un support intermédiaire d'un ou plusieurs godets destinés à recevoir chacun une composition différente. Le couvercle et la base présentent des parois latérales sensiblement perpendiculaires à la surface de distribution, et les compostions qui remplissent les godets ne sont pas en contact les unes avec les autres dans le boîtier.

Il existe dans le domaine des poudres cosmétiques de multiples méthodes pour créer des assemblages de teintes et de textures dans un même compartiment du boîtier.

Dans le domaine des cosmétiques coulés, il existe en revanche un nombre limité de méthodes pour créer des assemblages à l'intérieur d'un même compartiment de boîtier. Ainsi, le brevet US 6,058,942 a proposé un procédé de fabrication d'un produit cosmétique conditionné dans un boîtier qui consiste à déposer un premier matériau solide dans le récipient, à verser un deuxième matériau sous forme liquide dans le boîtier, puis à le laisser refroidir pour qu'il devienne solide. Le premier matériau a une température de fusion supérieure à celle du deuxième matériau, de manière à ne pas fondre lorsqu'il entre en contact le deuxième matériau fondu.

De la même manière, le brevet EP 1325692 assemble des produits anhydres coulés ou des poudres pressées avec une résine thermodurcissable. La résine est coulée, mais présente l'inconvénient de nécessiter un temps de durcissement de plusieurs heures. En outre, la résine ne peut pas être utilisée comme produit de maquillage ou de soin.

Dans le brevet FR 2956833, on a utilisé des moules silicone et plusieurs étapes de coulée pour créer des multi-teintes d'anhydres en strates. La surface de produit accessible à l'utilisateur est donc constituée d'une seule teinte. Ce produit ne peut pas offrir à l'utilisateur plusieurs teintes ou textures différentes de façon simultanée.

Enfin, des assemblages de poudres pressées avec des produits cosmétiques coulés n'ont pas été décrits.

### OBJECTIFS DE l'INVENTION

On souhaite développer des produits cosmétiques qui comportent dans un même conditionnement, voire dans un même récipient, plusieurs teintes et/ou plusieurs motifs de compositions moulées ou de cakes de poudres découpés au laser.

On souhaite également que l'assemblage n'utilise pas de godets et permette l'obtention d'un produit transportable dans lequel les teintes et les formes sont solidaires les unes des autres, et ne se détachent pas au cours du temps.

En suivant l'enseignement du brevet US 6,058,942, les inventeurs ont trouvé que le deuxième matériau que l'on coule autour du premier préalablement placé dans le récipient, peut se solidifier avant de remplir le récipient de façon uniforme et avant d'entourer totalement le premier matériau. Avec un deuxième matériau de point de fusion inférieur au premier, les inventeurs ont également observé qu'il n'est pas toujours possible de remplir complètement des interstices de petite taille.

Dans le cadre de l'invention, les inventeurs ont trouvé qu'il est possible de couler à chaud un produit cosmétique autour d'un ou plusieurs éléments cosmétiques, de formes, reliefs et teintes variées, afin de composer un assemblage solide fixé à un boîtier. Les éléments cosmétiques d'inclusion du produit de l'invention sont soit des mises en forme coulées soit des poudres pressées. Les inventeurs ont trouvé une composition de fixation qui épouse bien les formes d'inclusion, épouse bien la forme du récipient et remplit bien des interstices laissés entre les formes d'inclusion et les parois du boîtier, même lorsqu'ils sont étroits.

L'invention présente l'avantage d'utiliser un produit cosmétique pour assembler et fixer les produits cosmétiques d'inclusion. L'ensemble de l'assemblage est donc cosmétique. Par ailleurs, la vitesse de solidification du produit cosmétique utilisé pour la fixation est de l'ordre de quelques minutes ce qui rend la manipulation beaucoup plus aisée et le procédé de fabrication plus rapide.

L'invention est illustrée en référence aux Figures.

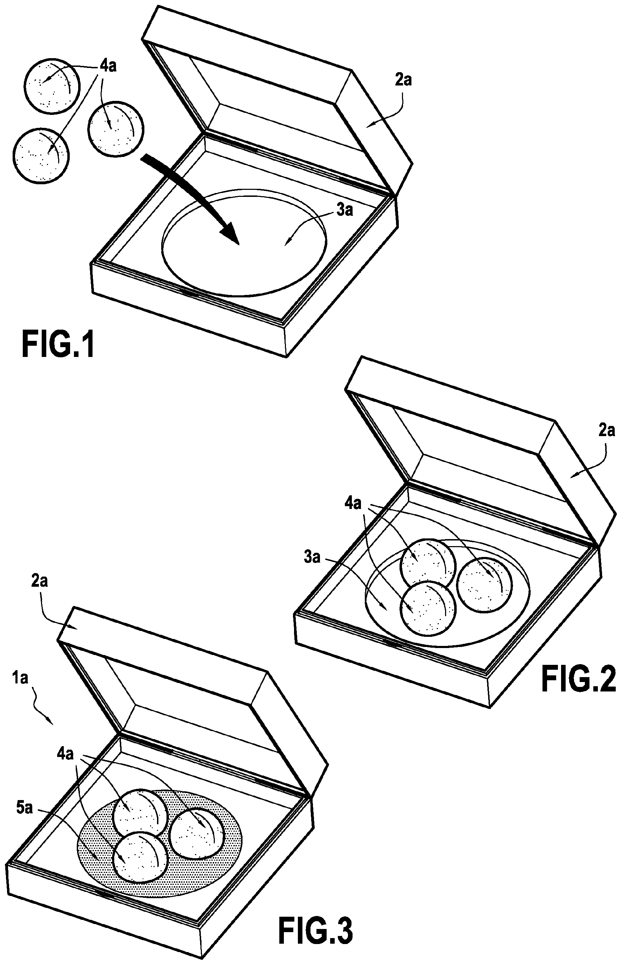

Les Figures 1 à 3 représentent une vue en perspective d'un assemblage selon l'invention obtenu à partir d'éléments d'inclusion sphériques qui ont été préalablement moulés, placés dans le fond d'un récipient comprenant un évidement circulaire, puis assemblés par coulage et solidification d'une composition de fixation contenant des cires, des huiles et des pâteux.

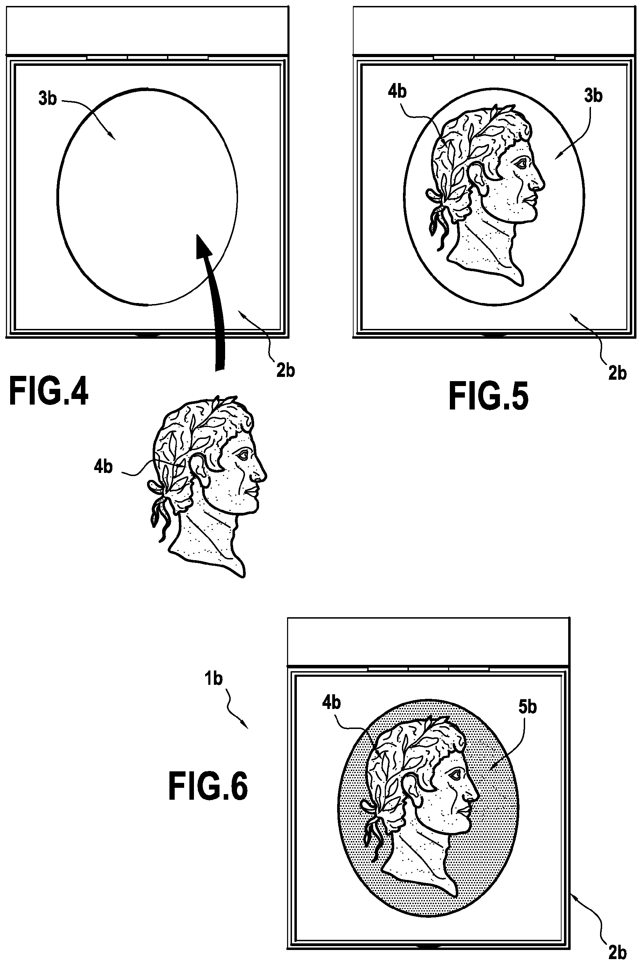

Les Figures 4 à 6 représentent un assemblage sous forme d'un camée comprenant une pièce d'inclusion qui a été moulée et placée dans un récipient comprenant un évidement oval, puis immobilisée par coulage et solidification d'une composition de fixation contenant des cires, des huiles et des pâteux.

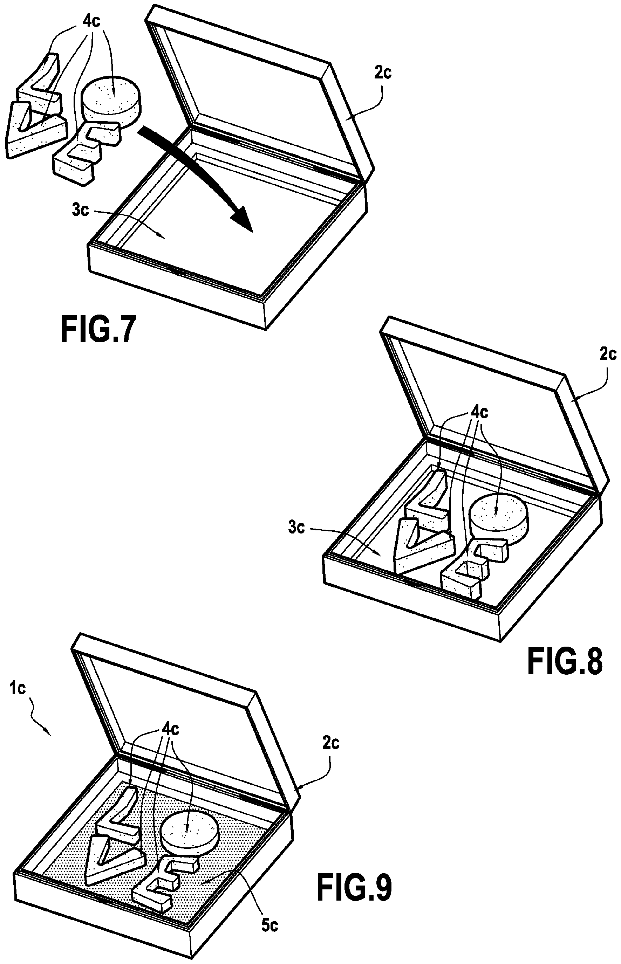

Les Figures 7 à 9 représentent une vue en perspective d'un assemblage de pièces d'inclusions de poudres pressées placées dans l'évidement rectangulaire d'un récipient. Les pièces d'inclusions sont rendues solidaires par coulage et solidification d'une composition de fixation sous forme de gel transparent nacré contenant des huiles gélifiées par un polymère.

### DESCRIPTION DE L'INVENTION

Selon un premier aspect, l'invention concerne produit cosmétique en relief comprenant un récipient doté d'au moins une cavité dans laquelle est disposé un assemblage d'au moins une pièce d'inclusion solide à 25°C et d'une composition de fixation solide à 25°C,
- la pièce d'inclusion et la composition de fixation étant en contact l'une avec l'autre et étant de compositions différentes,
caractérisé en ce que la pièce d'inclusion a une dureté supérieure à celle de la composition de fixation,
- la composition de fixation est dotée d'un point de goutte supérieur à 50°C et d'une dureté moyenne - mesurée à 25°C à l'aide d'un texturomètre muni d'une sonde hémisphérique - inférieure à 2 800 g, et en ce que
- la composition de fixation contient au moins une huile et un composé structurant.

Il a en effet été découvert dans le cadre de la présente invention, que le coulage d'un produit particulier autour des éléments cosmétiques moulés ou pressés en vue de les assembler dans un pack permet de les fixer solidement entre eux dans un même récipient, sans altérer leur structure, tout en adhérant bien aux surfaces du récipient, tout en recouvrant les surfaces du récipient de manière uniforme et tout en présentant une vitesse de refroidissement relativement rapide.

L'invention présente l'avantage de permettre l'assemblage de plusieurs pièces de produits cosmétiques qui ont été préalablement mises en forme de façon indépendante. Le produit de l'invention permet également la présentation et la solidarisation de plusieurs textures dans un même récipient, par exemple deux produits coulés de dureté différentes, ou un produit poudre et un produit coulé, sans avoir recours à l'utilisation de godets traditionnels que l'on vient fixer dans le boîtier.

Enfin, le produit de l'invention permet une grande adaptabilité à différentes formes de boîtiers sans avoir besoin de chercher un format de godet qui s'y adapte parfaitement ou sans avoir besoin d'adapter les formats de mise en forme des produits cosmétiques.

Le récipient du produit de l'invention peut revêtir toute forme telle que circulaire ou rectangulaire. Il comprend de préférence une seule cavité dont la forme est avantageusement indépendante de celle du récipient et qui peut être rectangulaire, circulaire ou ovale. Le récipient du produit de l'invention est avantageusement dépourvu de godet. La cavité est de préférence non compartimentée et son fond est plat, si bien qu'un liquide la remplit entièrement lorsqu'on l'y verse. Dans un mode de réalisation préféré, le récipient comprend une seule cavité et est dépourvu de godet.

Dans le récipient, la composition de fixation recouvre avantageusement toute la surface du fond de la cavité, et laisse de préférence affleurer la pièce d'inclusion.

Le produit de l'invention peut comprendre au moins une pièce d'inclusion, par exemple au moins deux. Les pièces d'inclusion peuvent être avantageusement de composition, de teinte et/ou de texture différentes.

Selon un mode de mise en oeuvre, la pièce d'inclusion a une dureté supérieure à celle de la composition de fixation, notamment lorsque la pièce d'inclusion contient majoritairement des corps gras tels que des huiles ou des cires.

Les pièces d'inclusion peuvent être anhydres, ou contenir une phase aqueuse et une phase grasse.

Les textures de type anhydre coulé utilisées pour la pièce d'inclusion sont de préférence des textures dures qui résistent au démoulage et qui conservent bien les formes potentiellement délicates d'un moule silicone. Un anhydre coulé pourra par exemple contenir de 10 à 15% en poids de cire(s), de 0 à 10% en poids de pâteux, et de 60 à 70% en poids d'huile(s).

La dureté des éléments d'inclusion - de préférence lorsqu'ils sont des anhydres coulés et moulés - est supérieure à la dureté de la composition de fixation. Par exemple, la dureté des éléments d'inclusion qui sont des anhydres coulés est supérieure ou égale à 2600 g, de préférence comprise entre 2 800 g et 4 500 g. La pièce d'inclusion peut avoir une dureté supérieure à celle de la composition de fixation.

Les textures de type émulsion solide moulée utilisées pour la pièce d'inclusion sont de préférence des émulsions eau-dans-huile.

Les textures de type poudre utilisées pour la pièce d'inclusion peuvent être compactées en outillage métal sans godet (ce qu'on appelle des cakes de poudre). Des motifs peuvent dans ce cas être obtenus avec l'outillage de compactage. On peut également obtenir les motifs en compactant de grandes surfaces de poudre et en effectuant une découpe laser des motifs choisis. Ce procédé a l'avantage d'éviter de concevoir un outillage spécifique pour des prototypes de motifs. Enfin, les poudres peuvent être mises en forme sous forme de slurrys. Les slurrys sont ensuite extrudés et découpés à l'emporte pièces. Les formes obtenues peuvent être plates ou légèrement en relief.

On pourra utiliser comme poudre compactée, un produit contenant 80 à 90% en poids d'un mélange de poudres choisies parmi des charges et des pigments, et 10 à 20% en poids d'huile(s) utilisée(s) comme liant des poudres.

Le produit cosmétique de l'invention peut être un produit de maquillage, un produit de soin, un produit de protection solaire ou un produit parfumant.

La pièce d'inclusion et la composition de fixation peuvent être indépendamment choisies parmi des produits de maquillage des lèvres, des yeux, des joues, des sourcils, du visage, et du corps, par exemple un rouge à lèvres, un fard à joues ou un fard à paupières.

Dans un mode de réalisation, la composition de fixation est un fard à paupières, un fard à joues, un gloss ou un rouge à lèvres. La pièce d'inclusion est par exemple un rouge à lèvres comprenant des cires, ou un fard à paupières sous forme de poudres pressées.

La pièce d'inclusion peut contenir des pigments minéraux, organiques ou nacrés, et éventuellement des charges. La composition de fixation peut contenir ou non des pigments.

Parmi les pigments minéraux, on peut citer, à titre d'exemples le dioxyde de titane, éventuellement traité en surface ; les oxydes de fer noir, jaune, rouge et brun ; le violet de manganèse ; le bleu outremer ; l'oxyde de chrome ; l'oxyde de chrome hydraté et le bleu ferrique.

Parmi les pigments organiques, on peut citer, par exemple, les pigments D & C red n° 19 ; D & C red n° 9 ; D & C red n° 21 ; D & C orange n° 4 ; D & C orange n° 5 ; D & C red n° 27 ; D & C red n° 13 ; D & C red n° 7 ; D & C red n° 6 ; D &C yellow n° 5 ; D & C red n° 36 ; D & C orange n° 10 ; D & C yellow n° 6 ; D & C red n° 30 ; D &C red n° 3 ; le noir de carbone et les laques à base de carmin de cochenille.

Les pigments nacrés peuvent être choisis notamment parmi les pigments nacrés blancs, tels que le mica recouvert d'oxyde de titane, l'oxychlorure de bismuth ; et les pigments nacrés colorés, tels que le mica titane avec des oxydes de fer, le mica titane avec du bleu ferrique ou de l'oxyde de chrome, le mica titane avec un pigment organique du type précité, ainsi que les pigments à base d'oxychlorure de bismuth.

Les charges peuvent être choisies parmi le talc, un silicate de magnésium hydraté; les micas de dimensions de 2 à 200 µm; le kaolin, un silicate d'aluminium hydraté; les oxydes de zinc et de titane ; le carbonate de calcium, le carbonate et l'hydrocarbonate de magnésium ; la silice ; le stéarate de zinc, de magnésium ou de lithium, le laurate de zinc, le myristate de magnésium ; les poudres de polymères synthétiques, tels que le polyéthylène, les polyesters et les polyamides (par exemple le nylon); la silice sphérique ; les dioxydes de titane sphériques; les billes de verre et de céramique ; des poudres de matériaux organiques d'origine naturelle comme les amidons de maïs, de blé, de riz, réticulées ou non ; des poudres de polymères de synthèse, réticulées ou non, sphéronisées, comme des poudres de polyamide telles que des poudres de poly-β-alanine et des poudres de nylon, des poudres d'acides polyacrylique ou polyméthacrylique, des poudres de polystyrène réticulées par le divinylbenzène, des poudres de résine de silicone, des poudres de téflon.

Le point de goutte de la composition de fixation est supérieur à 50°C, de préférence supérieur à 55°C, par exemple compris entre 55 et 60°C, compris entre 70 et 80°C, ou entre 75 et 85°C.

La composition de fixation a de préférence une dureté inférieure à 2 600 g. Sa dureté est de préférence supérieure à 600 g.

La composition de fixation a par exemple
- une dureté comprise entre 1 600 g et 2 600 g et un point de goutte compris entre 70 et 80°C, ou
- une dureté comprise entre 600 g et 1700 g et un point de goutte compris entre 75 et 85°C, ou
- une dureté comprise entre 1000 g et 1600 g et un point de goutte compris entre 55 et 60°C.

La dureté des compositions peut être mesurée avec un texturomètre de la marque STABLE MICRO SYSTEMS, modèle TA.XT.Plus, par pénétration dans la composition d'une sonde, par exemple une sonde hemisphérique, à une vitesse et une force imposées par l'appareil.

Le point de goutte peut être mesuré avec un appareil Mettler Toledo DP70 doté de cupules normalisées diamètre d'orifice 2,8 mm, en adoptant une vitesse de chauffe de 2,0°C/min, une température de départ de 40 °C, et une température de fin de 95 °C. La goutte est détectée visuellement à l'aide d'une caméra intégrée à l'appareil.

La composition de fixation contient un composé structurant qui peut être choisi parmi les cires et les polymères gélifiants d'huile. Lorsque la composition de fixation contient des cires, on préfère qu'elle en contienne peu de manière à ce que sa dureté ne soit pas trop élevée. D'une manière générale, on évitera d'incorporer plus de 10% en poids de cire(s) dans la composition. On pourra choisir d'incorporer des matières pâteuses pour structurer la composition de fixation sans augmenter sa dureté.

La composition de fixation est par exemple un gel anhydre contenant une huile et un agent gélifiant cette huile tel que décrit dans le document FR 2 958 159 ou FR 2 975 589. Un tel agent gélifiant est par exemple choisi parmi des polyamides, tels que des copolymères polyamides à terminaison ester (Uniclear®, Union Carbide), ou bien des polyamides siliconés, ou bien encore des dérivés de la L-Glutamide tels que le dibutyl lauroyl glutamide, commercialisé par Ajinomoto ou un de leurs mélanges, des silices pyrogénées, ou bien encore des copolymères comprenant au moins une unité styrène, hydrogénés ou non hydrogénés, tels que par exemple un copolymère hydrogéné styrène/méthylstyrène/indène, disponible dans le commerce.

Selon un mode de réalisation, l'agent gélifiant est un polymère de type polyamide à terminaison amide, de préférence un polymère de type polyamide à terminaison amide tertiaire (ATPA), de formule (I) ci-dessous : 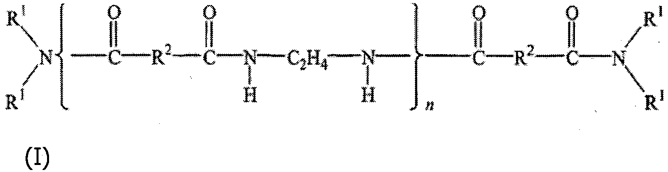 dans laquelle
- n est un nombre entier désignant le nombre d'unités présentes dans la formule (I), n pouvant varier de 1 à 10, préférentiellement de 1 à 5,
- les radicaux R¹, identiques ou différents, désignent un groupe hydrocarboné comprenant de 1 à 22 atomes de carbone ;
- les radicaux R², identiques ou différents à chaque occurrence, désignent un groupe hydrocarboné comprenant de 2 à 54 atomes de carbone, sous réserve qu'au moins 50% des radicaux R² aient de 30 à 42 atomes de carbone.

Un premier polymère préféré de type ATPA est un copolymère de dénomination (INCI) Ethylenediamine/Hydrogenated Dimer Dilinoleate Copolymer bis-di-C₁₄₋₁₈ Alkyl Amide, disponible dans le commerce.

Un deuxième polymère préféré de type ATPA est un copolymère de dénomination (INCI) Bis-Dioctadecylamide Dimer Dilinoleic Acid/Ethylenediamine Copolymer, disponible dans le commerce.

La composition selon l'invention comprend avantageusement de 10 à 50% en poids, plus préférentiellement de 15 à 30% en poids de la composition, d'un polymère de type ATPA.

On peut utiliser comme huile le mélange d'une huile non volatile de polyalkylène hydrogéné, notamment un polyisobutène hydrogéné et d'un ester d'acide gras comprenant au moins un groupe hydroxyl libre, par exemple un ester d'hydroxystéarate, de préférence l'hydroxystéarate d'éthylhexyle, lorsque l'agent gélifiant est un ATPA.

Dans un mode de réalisation particulier, la composition comprend avantageusement :
- de 15 à 30% en poids d'un polymère de type ATPA de dénomination (INCI) Bis-Dioctadecylamide Dimer Dilinoleic Acid/Ethylenediamine Copolymer,
- de 40 à 80% en poids de polyisobutène hydrogéné,
- de 5 à 20% en poids d' hydroxystéarate d'éthylhexyle, et de manière optionnelle :
- de 0 à 5% en poids d'alcool cétylique
- de 0 à 5 % d'un mélange de dibutyl lauroyl glutamide
- de 0 à 15% d'un copolymère hydrogéné
   styrène/méthylstyrène/indène.

La composition de fixation peut comprendre par exemple un mélange d'huile(s), de cire(s) et de pâteux. Par exemple, les huiles représentent entre 55 et 65% en poids de la composition, tandis que les cires représentent de 2 à 10% en poids de la composition et les pâteux représentent de 20 à 30% en poids de la composition.

Les huiles sont par exemple choisies parmi les esters, le polybutène, le polydécène et l'octyldodécanol. Les pâteux sont par exemple un polyisobutène, un C₁₀-C₃₀ ester, un copolymère d'oléfine ou un de leurs mélanges. On peut utiliser comme cire, une cire de polyéthylène, une cire d'abeilles, une cire d'ozokérite, ou un de leurs mélanges.

La composition de fixation peut être anhydre, ou contenir à la fois une phase aqueuse et une phase grasse. La composition de fixation peut être par exemple une émulsion eau-dans-huile, solide à température ambiante.

Le produit de l'invention peut comprendre l'assemblage d'une composition de fixation anhydre et de pièces d'inclusion anhydres, d'une composition de fixation anhydre et de pièces d'inclusion en émulsion eau-dans-huile, d'une composition de fixation en émulsion eau-dans-huile et de pièces d'inclusion anhydres, d'une composition de fixation en émulsion eau-dans-huile et de pièces d'inclusion en émulsion eau-dans-huile.

Dans un premier mode de réalisation particulier, le produit de l'invention comprend l'assemblage d'une composition de fixation sous la forme d'un gel anhydre comprenant du Bis-Dioctadécylamide Dimer Dilinoleic Acid/Ethylènediamine Copolymer et du Polyisobutène hydrogéné, et de pièces d'inclusion anhydres comprenant plus de 70% en poids de poudres.

Dans un mode de réalisation particulier, le produit de l'invention comprend l'assemblage d'une composition de fixation comprenant au moins un composé gras pâteux, des huiles et moins de 10% en poids de cires, et de pièces d'inclusion anhydres comprenant plus de 10% en poids de cires et plus de 50% en poids d'huiles.

Le produit de l'invention peut comprendre au moins un applicateur, sous forme d'un pinceau, d'une brosse ou d'embout en mousse fixé à une tige. L'applicateur peut être logé et encliqueter dans le récipient.

Selon un deuxième aspect, l'invention concerne un procédé de fabrication d'un produit cosmétique tel que décrit précédemment qui comprend la série d'étapes suivantes :
- mise en forme de la pièce d'inclusion,
- disposition de la pièce d'inclusion dans la cavité du récipient,
- chauffage de la composition de fixation à une température supérieure à son point de fusion, et coulage de ladite composition dans les espaces de la cavité du récipient laissés vides par la pièce d'inclusion,
- refroidissement de la composition de fixation jusqu'à solidification.

Une fois la composition de fixation versée dans le récipient, elle possède une surface en contact avec le récipient, une surface en contact avec la pièce d'inclusion et une surface libre. La surface libre de la composition de fixation est de préférence plane, tandis que la surface de la pièce d'inclusion sur laquelle l'utilisateur peut prélever du produit est plus haute que la surface libre de la composition de fixation. Par « plus haute », on entend plus haute par rapport au fond du récipient, si bien que la pièce d'inclusion fait saillie par rapport à la composition de fixation.

La pièce d'inclusion est généralement placée sur le fond du récipient sans entrer en contact avec les bords de celui-ci, de manière à ce que l'espace laissé libre entre la pièce d'inclusion et les bords du récipient soit rempli avec la composition de fixation. Dans le produit de l'invention, la composition de fixation peut donc entourer la base de la pièce d'inclusion de manière à l'immobiliser par rapport au récipient.

Dans le cas d'un anhydre coulé, la mise en forme de la pièce d'inclusion peut être réalisée par la série d'étapes suivantes : fusion d'un mélange comprenant majoritairement une phase grasse solide à 25°C, coulage dudit mélange dans un moule en silicone, refroidissement dudit mélange, puis démoulage de la pièce ainsi formée.

On peut utiliser des moules silicones pour obtenir différentes formes et reliefs. En utilisant des moules silicones, des produits cosmétiques coulés d'inclusion peuvent être en relief, les formes sont prononcées en trois dimensions et les motifs sont délicats ce qui rend le produit plus attrayant.

Dans les cas d'une poudre pressée, la mise en forme de la pièce d'inclusion peut être réalisée par la série d'étapes suivantes : compactage à l'aide d'une empreinte d'un mélange comprenant une proportion majoritaire de poudres, afin de former des cakes de poudre sans godet.

Dans les cas d'une poudre pressée, la mise en forme de la pièce d'inclusion peut aussi être réalisée par la série d'étapes suivantes : compactage d'un mélange comprenant une proportion majoritaire de poudres, et découpe laser selon la forme souhaitée.

La mise en forme de la pièce d'inclusion peut encore être réalisée par la série d'étapes suivantes : mélange de poudres dans un solvant afin d'obtenir une pâte qui est extrudée, découpage de la pâte à l'emporte-pièce selon la forme souhaitée, et séchage.

Selon le procédé de l'invention, la solidification de la surface de la composition de fixation s'effectue avantageusement en quelques secondes à quelques minutes à température ambiante (25°C).

La composition de fixation peut être coulée à une température inférieure ou égale à 99°C, plus particulièrement inférieure à 95°C.

Dans un mode de réalisation, le récipient a une température avant le coulage de la composition de fixation qui est égale à la température de coulage de la composition de fixation. Alternativement, le récipient a une température avant le coulage de la composition de fixation inférieure à la température de coulage de la composition de fixation, par exemple de l'ordre de 20 à 25°C.

Le récipient peut être un dispositif de conditionnement sous forme d'un boîtier comportant un fond définissant un premier compartiment creux pour recevoir l'assemblage de la pièce d'inclusion et de la composition de fixation. Le boîtier comprend un couvercle qui peut être articulé sur le fond et comporter un évidement dans le fond duquel est monté un miroir. Il peut être en matière plastique ou en métal.

Le fond peut définir un évidement dans lequel est disposé au moins un applicateur, telle qu'une mousse ou un pinceau.

Dans le produit cosmétique de l'invention, un seul compartiment comprend plusieurs compositions différentes. Au contraire, dans l'art antérieur, le fond du boîtier définit plusieurs compartiments destinés chacun à recevoir un produit différent, et dans chacun des compartiments sont disposées des godets ou des coupelles que l'on vient coller ou emboîter dans les compartiments, si bien que les compositions cosmétiques ne sont pas coulées ou compactées directement dans le boîtier.

L'invention ne concerne pas notamment les produits sous forme de sticks de rouge à lèvres ou de sticks de fond de teint bicolores qui ne comprennent pas un récipient à fond plat. Dans ces produits, la dureté des deux teintes sont équivalentes afin de garantir le dépôt du produit sur la peau sans cassure du raisin, ou usure prématurée d'une teinte par rapport à l'autre.

### DESCRIPTION DETAILLEE DES FIGURES

La Figure 3 représente une vue en perspective d'un produit cosmétique (1a) de maquillage selon l'invention. Dans les Figures 1 et 2, trois éléments d'inclusion sphériques (4a) qui ont été préalablement moulés et démoulés, sont placés sur le fond plan de la cavité (3a) circulaire d'un récipient (2a) de forme carrée. Ces éléments d'inclusion contiennent majoritairement des corps gras comprenant un mélange d'huiles et de cires. Dans la Figure 3, les éléments d'inclusion ont été assemblés les uns aux autres et rendus solidaires du récipient, par coulage dans la cavité (3a) d'une composition de fixation (5a) préalablement chauffée à 95°C. La composition de fixation une fois coulée se solidifie en quelques minutes, ce qui permet de manipuler le produit très rapidement. La composition de fixation contient des cires, des huiles et des pâteux ; sa dureté est inférieure à 2 800 g et son point de goutte est supérieur à 50°C. On choisit la taille relative des éléments d'inclusion et la hauteur de la cavité de manière à ce que les pièces d'inclusion affleurent de la surface de la composition de fixation qui remplit la cavité, et produise un produit cosmétique en relief.

La Figure 6 représente une vue en perspective d'un produit cosmétique (1b) parfumant selon l'invention. Dans les Figures 4 et 5, un élément d'inclusion sous la forme d'un buste (4b) qui a été préalablement moulé et démoulé, est placé sur le fond plan de la cavité (3b) ovale d'un récipient (2b) de forme carrée. Dans la Figure 6, le buste a été rendu solidaire du récipient par coulage d'une composition de fixation (5b) à 95°C de manière à remplir la cavité (3b).

La Figure 9 représente une vue en perspective d'un produit cosmétique (1c) de maquillage selon l'invention. Dans les Figures 7 et 8, quatre éléments d'inclusion sphériques (4c) qui ont été préalablement mises en forme par compactage, sont placés sur le fond plan de la cavité (3c) carrée d'un récipient (2c) de forme carrée. Ces éléments d'inclusion contiennent majoritairement des poudres qui ont été pressées. Dans la Figure 9, les éléments d'inclusion ont été assemblés les uns aux autres et rendus solidaires du récipient, par coulage dans la cavité (3c) d'une composition de fixation (5c) préalablement chauffée à 95°C. La composition de fixation une fois coulée se solidifie en quelques minutes, ce qui permet de manipuler le produit très rapidement. La composition de fixation est un gel transparent nacré contenant des huiles gélifiées par un polymère ; sa dureté est inférieure à 2 800 g et son point de goutte est supérieur à 50°C. On choisit la taille relative des éléments d'inclusion et la hauteur de la cavité de manière à ce que les pièces d'inclusion affleurent de la surface de la composition de fixation qui remplit la cavité, et produise un produit cosmétique en relief.

L'invention est illustrée par les exemples suivants.

### EXEMPLE: Assemblages d'éléments d'inclusion et d'une composition de fixation coulée

Chaque produit se compose d'au moins un élément d'inclusion, qui est déposé au fond d'une cavité formée dans un conditionnement unitaire, cavité dans laquelle on coule une composition de fixation. L'ensemble des éléments sont formés d'ingrédients cosmétiques, le tout formant une composition pour le maquillage, le soin ou la protection de la peau, notamment la peau du visage.

La dureté des compositions a été mesurée avec un texturomètre de la marque STABLE MICRO SYSTEMS, modèle TA.XT.Plus, par pénétration d'une sonde hémisphérique de diamètre 12.7 mm (appelée doigt Delrin). La mesure a été réalisée dans un pot Cléopâtre de 100 ml, et elle a été répétée trois fois.

Les paramètres de la mesure étaient les suivants :
- Approche 2 mm/s
- Test 2 mm/s
- Retrait 2 mm/s
- Pénétration 13 mm
- Relaxation 25 sec
- Force déclenchante : 2 g.

Le point de goutte a été mesuré avec un appareil Mettler Toledo DP70 doté de cupules normalisées diamètre d'orifice 2,8 mm, en adoptant une vitesse de chauffe de 2,0 °C/min, une température de départ de 40 °C, et une température de fin de 95 °C. La goutte est détectée visuellement à l'aide d'une caméra intégrée à l'appareil.

### 2.1 Eléments d'inclusion de Composition A

La composition A est utilisée pour la préparation d'éléments d'inclusion de type coulé. Les éléments cosmétiques « d'inclusion » de type coulé sont par exemple mis en forme par coulage dans des moules silicone.

La composition A présente la formule suivante (% en poids) :

| | % |
|---|---|
| Cires | 12,7 |
| Pâteux | 3,4 |
| Huiles | 64,5 |
| Charges | 15,9 |
| Autres | qsp 100 |

Les cires sont un mélange de polyéthylène, de cire d'abeille et d'ozokérite. Les pâteux sont constitués d'un mélange de Bentone et de C₁₀-C₃₀ esters. Les huiles sont constituées d'un mélange de polyisobutène, d'esters, de polydécène et d'octyldodécanol. Les charges sont constituées de pigments et de nacres dont la nature et la proportion respective varient en fonction de la teinte que l'on souhaite donner à la composition. La fraction « Autres » est constituée de conservateurs, agents actifs ou parfums.

La composition A présente un point de goutte de 78,2°C, une température de fusion de 77,3°C, et une dureté moyenne de 4424 g, mesurés selon les méthodes décrites précédemment.

### 2.2 Eléments d'inclusion de Composition B

On utilise la composition B pour la préparation d'éléments d'inclusion. Des éléments cosmétiques « d'inclusion » de type poudres sèches sont mis en forme par un procédé classique tel que par compactage, ou par extrusion, découpe à l'emporte-pièce et séchage.

On prépare une composition de type fard à paupière de formule suivante (% en poids) :

| | % |
|---|---|
| Talc traité diméthicone | 29,4 |
| Magnésium Stéarate | 2,0 |
| Nylon 12 | 3,0 |
| Calcium Sodium Borosilicate | 0,5 |
| Pigments | 0,4 |
| Nacres | 50,0 |
| Esters | 14,0 |
| Phénoxyéthanol | 0,7 |

On reproduit la même formule en variant la composition en pigment de façon à obtenir une teinte rose, marron ou beige.

### 2.3 Composition de fixation C

On prépare une composition de fixation, sous la forme d'un gel transparent. Ce gel peut être utilisé lui-même en tant que composition de fixation, ou bien être additionné d'une charge comprenant des pigments et/ou des nacres.

La formule du gel transparent est détaillée ci-dessous (% en poids) :

| | % |
|---|---|
| Phase A | |
| Bis-Dioctadécylamide Dimer Dilinoleic Acid/Ethylènediamine Copolymer | 21 |
| Ethylhexyl Hydroxystéarate | 11 |
| Polyisobutène hydrogéné | 56 |

| Phase B | |
|---|---|
| Copolymère styrène hydrogéné /méthylstyrène/indène | 10 |
| Alcool Cétylique | 3,2 |
| Dibutyl Lauroyl Glutamide | 0,4 |
| Dibutyl Ethylhexanoyl Glutamide | 0,4 |

### Procédé de préparation du gel

On fait fondre les différents composants de la phase A à 95°C sous agitation (Rayneri TurboTest, 300 tours/mn). On ajoute la phase B et on laisse fondre sous agitation.

On disperse 8% de nacres et 92% de la préparation obtenue précédemment. Les nacres sont dispersées de façon homogène dans la phase fondue A+B.

La composition C est un gel coloré présentant une dureté de 654 g selon la méthode décrite précédemment, et un point de goutte égal à 82,8°C.

### 2.4 Composition de fixation D

On utilise comme produit de fixation, une composition D de formule (% en poids) :

| | % |
|---|---|
| Cires | 10,2 |
| Pâteux | 25,5 |
| Huiles | 60,3 |
| Charges | 0,7 |
| Autres | qsp 100 |

Les cires sont un mélange de polyéthylène, de cire de paraffine et de cire d'ozokérite. Les pâteux sont constitués d'un mélange d'EPS copolymère, de Bentone et de polyisobutène hydrogéné. Les huiles sont constituées d'un mélange de polybutène, d'esters et d'octyldodécanol. Les charges sont constituées de pigments et de nacres dont la nature et la proportion respective varient en fonction de la teinte que l'on souhaite donner à la composition. La fraction « Autres » est constituée de conservateurs, agents actifs ou parfums.

La composition présente un point de goutte de 75,4°C et une dureté moyenne de 1867 g, mesurés selon les méthodes décrites précédemment.

### 2.5 Composition de fixation E

On prépare une composition de fixation sous la forme d'une émulsion eau-dans-huile solide de formule suivante. Le nom des ingrédients correspond à leur nom chimique ou à leur dénomination INCI (% en poids).

| | % |
|---|---|
| Cire de polyéthylène | 3 |
| Cire d'abeille | 3 |
| Glycérides hydrogénés | 3,7 |
| Hectorite | 0,8 |
| Esters | 5,45 |
| Huiles | 9,7 |
| Filtres solaires liquides | 7,5 |
| Pigments | 19 |
| Eau | qsp 100 |
| Emollients et conservateurs | 5,6 |
| Vinyl dimethicone/methicone silsesquioxane crosspolymer cellulose gum et polymethylsilsesquioxane | 4,6 |
| Filtres solaires solides | 1,5 |

On prépare la phase grasse en faisant fondre les cires puis en ajoutant à 85°C les pâteux et une partie des huiles à 85°C. On disperse ensuite l'hectorite puis les pigments dispersés préalablement le reste des huiles.

On prépare la phase aqueuse en chauffant l'eau et les émollients à 85°C, puis on y disperse le mélange Vinyl dimethicone/methicone silsesquioxane crosspolymer, cellulose gum et polymethylsilsesquioxane.

On verse la phase aqueuse dans la phase grasse à 85°C et on émulsionne pendant 15 min. On ajoute les conservateurs.

On mesure la dureté et le point de goutte selon les méthodes décrites précédemment :
- Dureté : 1302 g
- Point de goutte : 58,4°C

L'émulsion obtenue est utilisée comme agent de fixation des éléments moulés. On verse l'émulsion dans le récipient dans lequel on a préalablement placé les objets moulés.

### 2.6 Composition de fixation C bis

Une composition de fixation de l'invention a été préparée en utilisant les ingrédients suivants.

| Phase A | |
|---|---|
| Bis-Dioctadécylamide Dimer Dilinoleic Acid/Ethylènediamine Copolymer | 16,5 |
| Ethylhexyl Hydroxystéarate | 12 |
| Polyisobutène hydrogéné | 60,5 |

| Phase B | |
|---|---|
| Copolymère styrène hydrogéné /méthylstyrène/indène | 7,0 |
| Alcool Cétylique | 3,2 |
| Dibutyl Lauroyl Glutamide | 0,4 |
| Dibutyl Ethylhexanoyl Glutamide | 0,4 |

| | C bis |
|---|---|
| Dureté | 1643 g |
| Point de goutte | 83,4°C |

### 2.7 ASSEMBLAGE SELON l'INVENTION D'ELEMENTS D'INCLUSION ET DE COMPOSITIONS DE FIXATION

Les éléments d'inclusion ont été posés sur le fond d'un boîtier ou d'une coupelle, puis on a coulé le produit de fixation préalablement porté à une température de 95°C dans l'espace du compartiment ou de la coupelle laissé libre par les éléments d'inclusion. On a ensuite laisser refroidir la composition de fixation.

Les assemblages suivants ont été réalisés.

| **Assemblage** | **Eléments d'inclusions** | **Composition de fixation** | **Observation** |
|---|---|---|---|
| 1 | Composition A | Composition D | Figure 3 |
| 2 | Composition B | Composition C | Figure 9 |
| 3 | Composition A | Composition E | Figure 6 |
| 4 | Composition A | Composition C bis | - |

Les compositions obtenues sont solides et sont utilisées pour le maquillage de la peau.

On utilise indifféremment les compositions A et B pour préparer des pièces d'inclusion, et les compositions C, D, E et C bis comme produits de fixation, pour préparer des compositions cosmétiques dont l'usage peut être le maquillage ou bien le soin de la peau ou bien encore la protection de la peau quand celle-ci contient par exemple des filtres leur conférant une valeur de SPF.

La composition C bis a un point de goutte supérieur au point de goutte des inclusions A. Or, il est observé que, coulée à chaud à 95°C, la composition C bis occupe bien le fond du récipient de façon uniforme, entoure bien les inclusions, et remplit complètement les interstices de petite taille, notamment ceux de la figure 5. De plus, bien que la température de coulée soit supérieure à la température de fusion des inclusions A, les formes et les délimitations de celles-ci restent intactes. Ceci va à l'encontre de l'enseignement du brevet US 6,058,942.

## Revendications

1. Produit cosmétique en relief (1a, 1b, 1c) comprenant un récipient (2a, 2b, 2c) doté d'au moins une cavité (3a, 3b, 3c) dans laquelle est disposé un assemblage d'au moins une pièce d'inclusion solide à 25°C (4a, 4b, 4c) et d'une composition de fixation solide à 25°C (5a, 5b, 5c),
- la pièce d'inclusion (4a, 4b, 4c) et la composition de fixation (5a, 5b, 5c) étant en contact l'une avec l'autre et étant de compositions différentes,
**caractérisé en ce que** la pièce d'inclusion a une dureté supérieure à celle de la composition de fixation,
- la composition de fixation (5a, 5b, 5c) est dotée d'un point de goutte supérieur à 50°C et d'une dureté moyenne - mesurée à 25°C à l'aide d'un texturomètre muni d'une sonde hémisphérique - inférieure à 2 800 g, et **en ce que**
- la composition de fixation (5a, 5b, 5c) contient au moins une huile et un composé structurant.

2. Produit cosmétique selon la revendication 1, **caractérisée en ce que** le récipient (2a, 2b, 2c) comprend une seule cavité et **en ce qu'**il est dépourvu de godet.

3. Produit cosmétique selon les revendications 1 ou 2, **caractérisé en ce qu'**il comprend au moins deux pièces d'inclusion (4a, 4b, 4c).

4. Produit cosmétique selon l'une des revendications précédentes, **caractérisé en ce que** ledit produit est un produit de maquillage, un produit de soin, un produit de protection solaire ou un produit parfumant.

5. Produit cosmétique selon l'une des revendications précédentes, **caractérisé en ce que** la composition de fixation (5a, 5b, 5c) est un fard à paupières, un fard à joues, un gloss ou un rouge à lèvres.

6. Produit cosmétique selon l'une des revendications précédentes, **caractérisé en ce que** le composé structurant est choisi parmi les cires et les polymères gélifiants de l'huile.

7. Produit cosmétique selon l'une des revendications précédentes, **caractérisé en ce que** la pièce d'inclusion (4a, 4b, 4c) est un rouge à lèvres comprenant des cires, ou un fard à paupières sous forme de poudres pressées.

8. Produit cosmétique selon l'une des revendications précédentes, **caractérisé en ce que** la pièce d'inclusion (4a, 4b, 4c) fait saillie par rapport à la composition de fixation.

9. Produit cosmétique l'une des revendications précédentes, **caractérisé en ce que** la composition de fixation (5a, 5b, 5c) a une dureté comprise entre 1 600 g et 2 600 g et un point de goutte compris entre 70 et 80°C.

10. Produit cosmétique selon l'une des revendications 1 à 10, **caractérisé en ce que** la composition de fixation (5a, 5b, 5c) a une dureté comprise entre 600 g et 1700 g et un point de goutte compris entre 75 et 85°C.

11. Produit cosmétique selon l'une des revendications 1 à 10, **caractérisé en ce que** la composition de fixation (5a, 5b, 5c) a une dureté comprise entre 1000 g et 1600 g et un point de goutte compris entre 55 et 60°C.

12. Produit cosmétique selon l'une des revendications 1 à 11, **caractérisé en ce que** la composition de fixation est un gel anhydre contenant une huile et un agent gélifiant par exemple choisi parmi des polyamides, ou bien des polyamides siliconés, ou bien encore des dérivés de la L-glutamide ou un de leurs mélanges, des silices pyrogénées ou bien encore des copolymères comprenant au moins une unité styrène, hydrogénés ou non hydrogénés, tels que par exemple un copolymère hydrogéné styrène/méthylstyrène/indène.

13. Produit cosmétique selon la revendication 12, **caractérisé en ce que** l'agent gélifiant est un polymère de type polyamide à terminaison amide, de préférence un polymère de type polyamide à terminaison amide tertiaire (ATPA), de formule (I) ci- dessous : 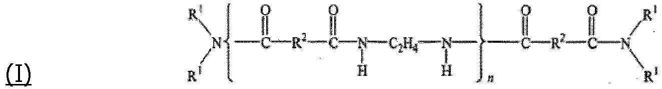 dans laquelle :
- n est un nombre entier désignant le nombre d'unités présentes dans la formule (I), n pouvant varier de 1 à 10, préférentiellement de 1 à 5,
- les radicaux R1, identiques ou différents, désignent un groupe hydrocarboné comprenant de 1 à 22 atomes de carbone ;
- les radicaux R2, identiques ou différents à chaque occurrence, désignent un groupe hydrocarboné comprenant de 2 à 54 atomes de carbone, sous réserve qu'au moins 50% des radicaux R2 aient de 30 à 42 atomes de carbone.

14. Produit cosmétique selon la revendication 13, caractérisé en ce le polymère de type ATPA est choisi parmi un premier copolymère de dénomination (INCI) Ethylenediamine/Hydrogenated Dimer Dilinoleate Copolymer bis-di-C₁₄₋₁₈ Alkyl Amide, et un deuxième copolymère de dénomination (INCI) Bis-Dioctadecylamide Dimer Dilinoleic Acid/Ethylenediamine Copolymer.

15. Produit cosmétique selon la revendication 13 ou 14, **caractérisé en ce que** la composition de fixation comprend de 10 à 50% en poids, plus préférentiellement de 15 à 30% en poids de la composition, d'un polymère de type ATPA.

16. Produit cosmétique selon l'une des revendications 12 à 15, **caractérisé en ce que** l'huile est un mélange d'une huile non volatile de polyalkylène hydrogéné, notamment un polyisobutène hydrogéné et d'un ester d'acide gras comprenant au moins un groupe hydroxyl libre, par exemple un ester d'hydroxystéarate, de préférence l'hydroxystéarate d'éthylhexyle, lorsque l'agent gélifiant est un ATPA.

17. Produit cosmétique selon l'une des revendications 12 à 16, **caractérisé en ce que** la composition de fixation comprend :
- de 15 à 30% en poids d'un polymère de type ATPA de dénomination (INCI) Bis-Dioctadecylamide Dimer Dilinoleic Acid/Ethylenediamine Copolymer,
- de 40 à 80% en poids de polyisobutène hydrogéné,
- de 5 à 20% en poids d' hydroxystéarate d'éthylhexyle, et de manière optionnelle :
- de 0 à 5% en poids d'alcool cétylique
- de 0 à 5 % d'un mélange de dibutyl lauroyl glutamide
- de 0 à 15% d'un copolymère hydrogéné styrène/méthylstyrène/indène.

18. Produit cosmétique selon l'une des revendications 1 à 11, **caractérisé en ce que** la composition de fixation comprend un mélange d'huile(s), de cire(s) et de pâteux ; par exemple les huiles représentent entre 55 et 65% en poids de la composition, tandis que les cires représentent de 2 à 10% en poids de la composition et les pâteux représentent de 20 à 30% en poids de la composition.

19. Produit cosmétique selon la revendication 18, **caractérisé en ce que** les huiles sont choisies parmi les esters, le polybutène, le polydécène et l'octyldodécanol ; les pâteux sont un polyisobutène, un C₁₀-C₃₀ ester, un copolymère d'oléfine ou un de leurs mélanges ; la cire est choisie parmi une cire de polyéthylène, une cire d'abeilles, une cire d'ozokérite, ou un de leurs mélanges.

20. Produit cosmétique selon l'une des revendications 1 à 17, **caractérisé en ce qu'**il comprend l'assemblage d'une composition de fixation sous la forme d'un gel anhydre comprenant du Bis-Dioctadécylamide Dimer Dilinoleic Acid/Ethylènediamine Copolymer et du Polyisobutène hydrogéné, et de pièces d'inclusion anhydres comprenant plus de 70% en poids de poudres.

21. Produit cosmétique selon l'une des revendications 1 à 11 et 18 à 19, **caractérisé en ce qu'**il comprend l'assemblage d'une composition de fixation comprenant au moins un composé gras pâteux, des huiles et moins de 10% en poids de cires, et de pièces d'inclusion anhydres comprenant plus de 10% en poids de cires et plus de 50% en poids d'huiles.

## Patentansprüche

1. Kosmetikprodukt mit Reliefstruktur (1a, 1b, 1c), umfassend einen Behälter (2a, 2b, 2c), der mit zumindest einem Hohlraum (3a, 3b, 3C) versehen ist, in welchem eine Anordnung zumindest eines bei 25 °C festen Einschlussstückes (4a, 4b, 4c) und einer bei 25 °C festen Fixierzusammensetzung (5a, 5b, 5c) angeordnet ist,
- wobei das Einschlussstück (4a, 4b, 4c) und die Fixierzusammensetzung (5a, 5b, 5c) miteinander in Kontakt stehen und unterschiedliche Zusammensetzungen sind, **dadurch gekennzeichnet, dass** das Einschlussstück eine größere Härte aufweist als jene der Fixierzusammensetzung,
- die Fixierzusammensetzung (5a, 5b, 5c) einen Tropfpunkt über 50 °C und einer mittleren Härte - gemessen bei 25 °C mithilfe eines Texturmeters mit halbkugelförmiger Sonde - unter 2.800 g aufweist, und dass
- die Fixierzusammensetzung (5a, 5b, 5c) zumindest ein Öl und eine strukturgebende Verbindung enthält.

2. Kosmetikprodukt nach Anspruch 1, **dadurch gekennzeichnet, dass** der Behälter (2a, 2b, 2c) einen einzigen Hohlraum umfasst und dass er keinen Tiegel aufweist.

3. Kosmetikprodukt nach den Ansprüchen 1 oder 2, **dadurch gekennzeichnet, dass** es zumindest zwei Einschlussstücke (4a, 4b, 4c) umfasst.

4. Kosmetikprodukt nach einem der vorhergehenden Ansprüche, **dadurch gekennzeichnet, dass** das Produkt ein Schminkprodukt, ein Pflegeprodukt, ein Sonnenschutzprodukt oder ein Duftprodukt ist.

5. Kosmetikprodukt nach einem der vorhergehenden Ansprüche, **dadurch gekennzeichnet, dass** die Fixierzusammensetzung (5a, 5b, 5c) ein Lidschatten, ein Rouge, ein Gloss oder ein Lippenstift ist.

6. Kosmetikprodukt nach einem der vorhergehenden Ansprüche, **dadurch gekennzeichnet, dass** die strukturgebende Verbindung ausgewählt ist aus Wachsen und gelbildenden Polymeren auf Ölbasis.

7. Kosmetikprodukt nach einem der vorhergehenden Ansprüche, **dadurch gekennzeichnet, dass** das Einschlussstück (4a, 4b, 4c) ein Lippenstift umfassend Wachse oder ein Lidschatten in Form von gepressten Pudern ist.

8. Kosmetikprodukt nach einem der vorhergehenden Ansprüche, **dadurch gekennzeichnet, dass** das Einschlussstück (4a, 4b, 4c) relativ zu der Fixierzusammensetzung herausragt.

9. Kosmetikprodukt nach einem der vorhergehenden Ansprüche, **dadurch gekennzeichnet, dass** die Fixierzusammensetzung (5a, 5b, 5c) eine Härte zwischen 1.600 g und 2.600 g und einen Tropfpunkt zwischen 70 und 80 °C aufweist.

10. Kosmetikprodukt nach einem der Ansprüche 1 bis 10, **dadurch gekennzeichnet, dass** die Fixierzusammensetzung (5a, 5b, 5c) eine Härte zwischen 600 g und 1.700 g und einen Tropfpunkt zwischen 75 und 85 °C aufweist.

11. Kosmetikprodukt nach einem der Ansprüche 1 bis 10, **dadurch gekennzeichnet, dass** die Fixierzusammensetzung (5a, 5b, 5c) eine Härte zwischen 1.000 g und 1.600 g und einen Tropfpunkt zwischen 55 und 60 °C aufweist.

12. Kosmetikprodukt nach einem der Ansprüche 1 bis 11, **dadurch gekennzeichnet, dass** die Fixierzusammensetzung ein wasserfreies Gel ist, das ein Öl und ein Geliermittel enthält, ausgewählt beispielsweise aus Polyamiden oder Silikonpolyamiden oder auch L-Glutamidderivaten oder einer ihrer Mischungen, pyrogenen Kieselsäuren oder auch Copolymeren, die mindestens eine Styroleinheit umfassen, hydriert oder unhydriert, wie beispielsweise ein hydriertes Styrol/Methylstyrol/Inden-Copolymer.

13. Kosmetikprodukt nach Anspruch 12, **dadurch gekennzeichnet, dass** das Geliermittel ein Polymer vom Typ eines mit Amid terminierten Polyamids ist, vorzugsweise ein Polymer vom Typ eines mit tertiärem Amid terminierten Polyamids (ATPA) der folgenden Formel (I): 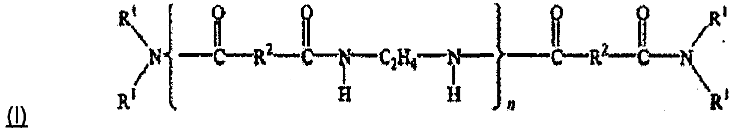 wobei:
- n eine ganze Zahl ist, welche die Anzahl der in der Formel (I) vorliegenden Einheiten bezeichnet, wobei n von 1 bis 10, vorzugsweise von 1 bis 5 variieren kann,
- die Reste R1, die identisch oder verschieden sind, eine Kohlenwasserstoffgruppe umfassend 1 bis 22 Kohlenstoffatome bezeichnen,
- die Reste R2, die bei jedem Auftreten identisch oder verschieden sind, eine Kohlenwasserstoffgruppe umfassend 2 bis 54 Kohlenstoffatome bezeichnen, mit der Maßgabe, dass zumindest 50 % der Reste R2 30 bis 42 Kohlenstoffatome aufweisen.

14. Kosmetikprodukt nach Anspruch 13, **dadurch gekennzeichnet, dass** das Polymer vom Typ ATPA ausgewählt ist aus einem ersten Copolymer der Bezeichnung (nach INCI) *Ethylenediamine* / *Hydrogenated Dimer Dilinoleate Copolymer bis-di-C₁₄₋₁₈ Alkyl Amide,* und einem zweiten Copolymer der Bezeichnung (nach INCI) *Bis-Dioctadecylamide Dimer Dilinoleic Acid* / *Ethylenediamine Copolymer.*

15. Kosmetikprodukt nach Anspruch 13 oder 14, **dadurch gekennzeichnet, dass** die Fixierzusammensetzung 10 bis 50 Gew.-%, noch bevorzugter 15 bis 30 Gew.-% der Zusammensetzung, eines Polymers vom Typ ATPA umfasst.

16. Kosmetikprodukt nach einem der Ansprüche 12 bis 15, **dadurch gekennzeichnet, dass** das Öl eine Mischung aus einem nichtflüchtigen, hydrierten Polyalkylenöl, insbesondere einem hydrierten Polyisobuten und einem Fettsäureester umfassend mindestens eine freie Hydroxylgruppe, beispielsweise einen Hydroxystearatester, vorzugsweise Ethylhexylhydroxystearat, ist, wenn das Geliermittel ein ATPA ist.

17. Kosmetikprodukt nach einem der Ansprüche 12 bis 16, **dadurch gekennzeichnet, dass** die Fixierzusammensetzung umfasst:
- 15 bis 30 Gew.-% eines Polymers vom Typ ATPA der Bezeichnung (nach INCI) *Bis-Dioctadecylamide Dimer Dilinoleic Acid* / *Ethylenediamine Copolymer,*
- 40 bis 80 Gew.-% hydriertes Polyisobuten,
- 5 bis 20 Gew.-% Ethylhexylhydroxystearat, und optional:
- 0 bis 5 Gew.-% Ketylalkohol,
- 0 bis 5 % einer Mischung aus Dibutyllauroylglutamid
- 0 bis 15 % eines hydrierten Styrol/Methylstyrol/Inden-Copolymers.

18. Kosmetikprodukt nach einem der Ansprüche 1 bis 11, **dadurch gekennzeichnet, dass** die Fixierzusammensetzung eine Mischung aus Öl(en), Wachs(en) und Pasten umfasst, wobei zum Beispiel die Öle zwischen 55 und 65 Gew.-% der Zusammensetzung darstellen, während die Wachse 2 bis 10 Gew.-% der Zusammensetzung darstellen und die Pasten 20 bis 30 Gew.-% der Zusammensetzung darstellen.

19. Kosmetikprodukt nach Anspruch 18, **dadurch gekennzeichnet, dass** die Öle ausgewählt sind aus den Estern, Polybuten, Polydeken und Oktyldekanol, die Pasten ein Polyisobuten, ein C₁₀-C₃₀-Ester, ein Olefin-Copolymer oder eine ihrer Mischungen sind, das Wachs ausgewählt ist aus einem Polyethylenwachs, einem Bienenwachs, einem Ozokeritwachs oder einer ihrer Mischungen.

20. Kosmetikprodukt nach einem der Ansprüche 1 bis 17, **dadurch gekennzeichnet, dass** es die Anordnung einer Fixierzusammensetzung in Form eines wasserfreien Gels umfassend Bis-Dioktadecylamid-Dimer-Dilinolsäure-/Ethylendiamin-Copolymer und Polyisobutenhydrogen und wasserfreie Einschlusstücke umfassend mehr als 70 Gew.-% Puder umfasst.

21. Kosmetikprodukt nach einem der Ansprüche 1 bis 11 und 18 bis 19, **dadurch gekennzeichnet, dass** es die Anordnung einer Fixierzusammensetzung umfassend zumindest eine pastöse Fettverbindung, Öle und weniger als 10 Gew.-% Wachse, und wasserfreie Einschlussstücke umfassend mehr als 10 Gew.-% Wachse und mehr als 50 Gew.-% Öle umfasst.

## Claims

1. In relief cosmetic product (1a, 1b, 1c) comprising a container (2a, 2b, 2c) having at least one cavity (3a, 3b, 3c) in which is positioned an assembly of at least one inclusion part which is solid at 25°C (4a, 4b, 4c) and of a fixing composition which is solid at 25°C (5a, 5b, 5c),:
- the inclusion part (4a, 4b, 4c) and the fixing composition (5a, 5b, 5c) being in contact with one another and having different compositions, **characterized in that** the hardness of the inclusion part is greater than the hardness of the fixing composition,
- the fixing composition (5a, 5b, 5c) has a drop point greater than 50°C and a mean hardness, measured at 25°C using a texture analyser provided with a hemispheric probe, of less than 2800 g, and
- the fixing composition (5a, 5b, 5c) comprises at least one oil and one structuring compound.

2. Cosmetic product according to Claim 1, **characterized in that** the container (2a, 2b, 2c) comprises just one cavity and **in that** it is pot-free.

3. Cosmetic product according to Claim 1 or Claim 2, **characterized in that** it comprises at least two inclusion parts (4a, 4b, 4c).

4. Cosmetic product according to one of the preceding claims, **characterized in that** the said product is a make-up product, a care product, a sun protection product or a scenting product.

5. Cosmetic product according to one of the preceding claims, **characterized in that** the fixing composition (5a, 5b, 5c) is an eye shadow, a blusher, a gloss or a lipstick.

6. Cosmetic product according to one of the preceding claims, **characterized in that** the structuring compound is chosen from waxes and oil-gelling polymers.

7. Cosmetic product according to one of the preceding claims, **characterized in that** the inclusion part (4a, 4b, 4c) is a lipstick comprising waxes or an eye shadow in the form of pressed powders.

8. Cosmetic product according to one of the preceding claims, **characterized in that** the inclusion part (4a, 4b, 4c) projects with respect to the fixing composition.

9. Cosmetic product according to one of the preceding claims, **characterized in that** the fixing composition (5a, 5b, 5c) has a hardness of between 1600 g and 2600 g and a drop point of between 70 and 80°C.

10. Cosmetic product according to one of Claims 1 to 10, **characterized in that** the fixing composition (5a, 5b, 5c) has a hardness of between 600 g and 1700 g and a drop point of between 75 and 85°C.

11. Cosmetic product according to one of Claims 1 to 10, **characterized in that** the fixing composition (5a, 5b, 5c) has a hardness of between 1000 g and 1600 g and a drop point of between 55 and 60°C.

12. Cosmetic product according to one of Claims 1 to 11, **characterized in that** the fixing composition is an anhydrous gel comprising an oil and a gelling agent, for example chosen from polyamides, or silicone polyamides, or else L-glutamide derivatives, or one of their mixtures, fumed silicas, or else hydrogenated or nonhydrogenated copolymers comprising at least one styrene unit, such as, for example, a hydrogenated styrene/methylstyrene/indene copolymer.

13. Cosmetic product according to Claim 12, **characterized in that** the gelling agent is a polymer of polyamide type comprising an amide ending, preferably a polymer of polyamide type comprising a tertiary amide ending (ATPA), of formula (I) below: 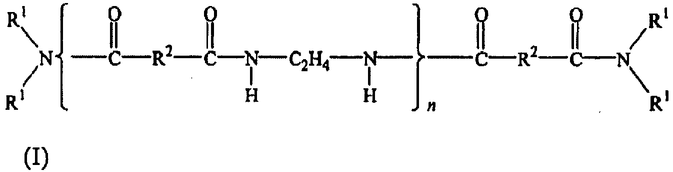 in which:
- n is an integer denoting the number of units present in the formula (I), it being possible for n to vary from 1 to 10, preferably from 1 to 5,
- the R¹ radicals, which are identical or different, denote a hydrocarbon group comprising from 1 to 22 carbon atoms,
- the R² radicals, which are identical or different in each instance, denote a hydrocarbon group comprising from 2 to 54 carbon atoms, with the proviso that at least 50% of the R² radicals have from 30 to 42 carbon atoms.

14. Cosmetic product according to Claim 13, **characterized in that** the polymer of ATPA type is selected from a first copolymer having the name (INCI) Ethylenediamine/Hydrogenated Dimer Dilinoleate Copolymer bis-di-C₁₄₋₁₈Alkyl Amide, and from a second copolymer having the name (INCI) Bis-Dioctadecylamide Dimer Dilinoleic Acid/Ethylenediamine Copolymer.

15. Cosmetic product according to Claim 13 or 14, **characterized in that** the fixing composition comprises from 10% to 50% by weight of the composition, more preferably from 15% to 30% by weight of the composition, of a polymer of ATPA type.

16. Cosmetic product according to one of Claims 12 to 15, **characterized in that** the oil is a mixture of non-volatile hydrogenated polyalkylene oil, in particular a hydrogenated polyisobutene, and of a fatty acid ester comprising at least one free hydroxyl group, for example a hydroxystearate ester, preferably ethylhexyl hydroxystearate, when the gelling agent is an ATPA.

17. Cosmetic product according to one of Claims 12 to 16, **characterized in that** the fixing composition comprises :
- from 15% to 30% by weight of a polymer of ATPA type having the name (INCI) Bis-Dioctadecylamide Dimer Dilinoleic Acid/Ethylenediamine Copolymer,
- from 40% to 80% by weight of hydrogenated polyisobutene,
- from 5% to 20% by weight of ethylhexyl hydroxystearate and, optionally:
- from 0% to 5% by weight of cetyl alcohol,
- from 0% to 5% of a dibutyl lauroyl glutamide mixture,
- from 0% to 15% of a hydrogenated styrene/methylstyrene/indene copolymer.

18. Cosmetic product according to one of Claims 1 to 11, **characterized in that** the fixing composition comprise a mixture of oil(s), wax(es) and paste(s) ; for example, the oils represent between 55% and 65% by weight of the composition, while the waxes represent from 2% to 10% by weight of the composition and the pastes represent from 20% to 30% by weight of the composition.

19. Cosmetic product according to Claim 18, characterized the oils are chosen from esters, polybutene, polydecene and octyldodecanol; the pastes are a polyisobutene, a C₁₀-C₃₀ ester, an olefin copolymer or one of their mixtures; the wax is selected from polyethylene wax, a beeswax, an ozokerite wax or one of their mixtures.

20. Cosmetic product according to one of Claims 1 to 17, **characterized in that** it comprises the assembly of a fixing composition in the form of an anhydrous gel comprising Bis-Dioctadecylamide Dimer Dilinoleic Acid/Ethylenediamine Copolymer and hydrogenated polyisobutene, and anhydrous inclusion parts comprising more than 70% by weight of powders.

21. Cosmetic product according to one of Claims 1 to 11 and 18 to 19, **characterized in that** it comprises the assembly of a fixing composition comprising at least one pasty fatty compound, oils and less than 10% by weight of waxes, and anhydrous inclusion parts comprising more than 10% by weight of waxes and more than 50% by weight of oils.
